(19) Europäisches Patentamt
European Patent Office
Office européen des brevets

(11) Publication number: 0 089 674
B1

(12) EUROPEAN PATENT SPECIFICATION

(45) Date of publication of patent specification: 27.12.85

(21) Application number: 83102809.7

(22) Date of filing: 22.03.83

(51) Int. Cl.$^4$: C 07 C 59/29, C 07 C 59/245, C 07 C 59/31, C 07 C 57/26, C 07 F 9/54, C 07 C 69/608, C 07 C 33/20, C 07 C 69/675, A 61 K 31/19

# (54) Pentanedioic acid derivatives.

(30) Priority: 22.03.82 US 360543

(43) Date of publication of application: 28.09.83 Bulletin 83/39

(45) Publication of the grant of the patent: 27.12.85 Bulletin 85/52

(84) Designated Contracting States: BE CH DE FR GB IT LI NL SE

(56) References cited:
FR-A-2 244 485
US-A-3 112 249
US-A-3 629 449
US-A-3 818 080

JOURNAL OF PHARMACEUTICAL SCIENCES, vol. 69, no. 5, May 1980, pages 506-509, American Pharmaceutical Association, M.R. BOOTS et al.: "Hypocholesterolemic agents VII: Inhibition of beta-hydroxy-beta-methylglutaryl-CoA reductase by monoesters of substituted glutaric acids"

(73) Proprietor: G.D. Searle & Co.
Box 1045
Skokie, Illinois 60076 (US)

(72) Inventor: Lowrie, Harman Smith
3615 Central Road
Glenview Illinois (US)
Inventor: Baran, John Stanislaus
659 Locust Street
Winnetka, Ill. (US)

(74) Representative: Beil, Walter, Dr. et al
BEIL, WOLFF & BEIL Rechtsanwälte
Adelonstrasse 58
D-6230 Frankfurt am Main 80 (DE)

(56) References cited:
JOURNAL OF CHROMATOGRAPHY, vol. 164, 1979, pages 319-329, Elsevier Scientific Publishing Company, Amsterdam, NL. M. SPITELLER et al.: "Über das Auftreten alpha-Alkyl-substituierter Äpfelsäuren beta-Hydroxy-beta-Alkyl-substituierter Dicarbon und Tricarbonsäurederivate als normale Stoffwechselprodukte"

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 089 674 B1

**Description**

Background of the invention

The invention relates to novel pentanedioic acid derivatives. In particular, it relates to novel pentanedioic acid derivatives of formula I which are useful to inhibit the formation of serum cholesterol. The novel compounds exhibit this utility by virtue of their ability to inhibit the activity of β-hydroxy-β-methyl-glutaryl coenzyme A (HMG CoA) and thus inhibit the formation of serum cholesterol. HMG CoA is a substance which controls the rate at which cholesterol is synthesized in mammalian liver (one of the two principal *in vivo* sources of serum cholesterol). Thus the compounds of the instant invention are useful to inhibit sterol biosynthisis in individuals predisposed to familial type hypercholesterolemia. The significance of such compounds is widely recognized, e.g. Breslow *et al.* Biochim. Biophys. Acta. *398,* 10(1975); Betheridge et al., Brit Med. J., *4,* 500 (1975); and Brown et al., J. Biol. Chem. *249,* 7306 (1974).

Prior art

The use of agents which lower serum cholesterol are widely described in the art as described above. Several pentanedioic acid derivatives are known in the art. For example, US—A—3 818 080 describes certain compounds useful for their antiulcerogenic activity, namely 3-hydroxy-3-ethylglutaric acid, 3-hydroxy-3-n-propylglutaric acid, 3-hydroxy-3-isopropylglutaric acid, 3-hydroxy-3-n-butylglutaric acid, 3-hydroxy-3-t-butylglutaric acid, 3-hydroxy-3-n-decylglutaric acid, 3-hydroxy-3-pentadecylglutaric acid and 3-hydroxy-3-cyclopentylglutaric acid. Said compounds have been disclaimed from the present claims as compounds of the formula I wherein $R_1$ and $R_2$ are both hydrogen and the radical

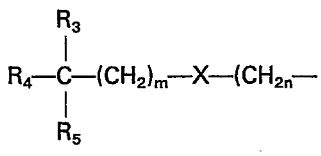

is ethyl, n-propyl, isopropyl, n-butyl, t-butyl, n-decyl, pentadecyl or cyclopentylmethyl.

From Journal to Pharmaceutical Sciences, Vol. 69, No. 5, May 1980, pages 506—509, American Pharmaceutical Association, M. R. Boots et al.: "Hypocholesterolemic Agents VII: Inhibition of 6-Hydroxy-β-methylglutaryl-CoA Reductase by Monoesters of Substituted Glutaric Acids", there are known 3-hydroxy - 3 - n - propylglutaric acid, 3-hydroxy - 3-isopropylglutaric acid, 3-hydroxy-3-n - butylglutaric acid and 3 - hydroxy - 3 - isobutylglutaric acid as intermediates for the preparation of certain 1-(4-biphenylyl) - n - pentyl hydrogen 3 - hydroxy - 3 - alkylglutarates. Said compounds have been disclaimed from the present claims as compounds of the formula I wherein $R_1$ and $R_2$ are both hydrogen and the radical

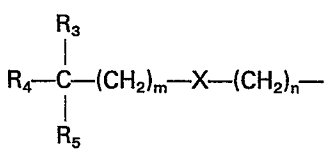

is n-propyl, isopropyl, n-butyl or isobutyl.

From Journal of Chromatographie, Vol. 164, 1979, pages 319—329, Elsevier Scientific Publishing Company, Amsterdam, NL; M. Spiteller et al.: "Über das Auftreten α-alkylsubstituierter Äpfelsäuren und β - hydroxy - β - alkyl - substituierter Dicarbon- und Tricarbonsäurederivate als normale Stoffwechselprodukte", there are known the dimethylesters of 3 - hydroxy - 3 - propylglutaric acid and 3 - hydroxy - 3 - isopropylglutaric acid as products of the normal metabolism. Said dimethylesters also have been disclaimed from the present claims.

From US—A—3 112 249 3-hydroxy-3-methylglutaric acid is known as degradation product of cholesterol. Said compound is not claimed in the present claims.

From US—A—3 629 449 the use of 3-hydroxy-3-methylglutaric acid for reducing serum cholesterol and blood lipid levels in warm-blooded animals is known. Said compound, however, is not claimed in the present claims.

From FR—A—2 244 485 the use of glutaric acid for treating psychosis is known. 3-Hydroxy-3-alkylglutaric acids are not mentioned in said patent application.

Summary of the invention

The present invention particularly provides compounds of formula 1:

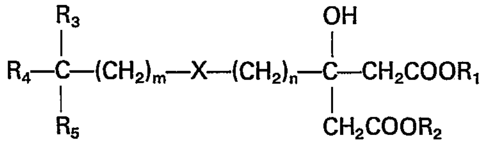

wherein $R_1$ and $R_2$ are:
a) hydrogen; or
b) alkyl of from 1 to 6 carbon atoms, inclusive;
$R^1$ and $R^2$ each being the same or different;
wherein $R_3$, $R_4$, and $R_5$ are:
a) hydrogen;
b) alkyl of from one to six carbon atoms, inclusive;
c) cycloalkyl, bicycloalkyl or polycycloalkyl of from 3 to 10 carbon atoms, inclusive;
$R_3$, $R_4$, and $R_5$ each being the same or different; or
wherein any two or all three of $R_3$, $R_4$, and $R_5$ are taken together to form a saturated hydrocarbon ring, having from 3 to 10 carbon atoms, inclusive;
wherein m is an integer from zero to 15, inclusive;
wherein n is an integer from zero to 15, inclusive;
wherein X is:
a) $—CH_2—$;
b) branched alkylene chain of from 2 to 15 carbon atoms; or
c) Cycloalkylene, bicycloalkylene, or polycycloalkylene of from 3 to 10 carbon atoms, inclusive; or
d) —O—
with the exception of those compounds of the formula wherein $R_1$ and $R_2$ are both hydrogen and the radical

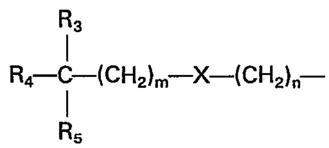

is ethyl, n-propyl isopropyl, n-butyl, t-butyl, isobutyl, n-decyl, pentadecyl or cyclopentylmethyl, and with the exception of the dimethylesters of 3-hydroxy-3-propylglutaric acid and 3-hydroxy - 3 - isopropylglutaric acid.

Examples of alkyls of from one to six carbon atoms, inclusive are methyl, ethyl, propyl, butyl, pentyl and hexyl, and the isomeric forms thereof.

Examples of cycloalkyl of from 3 to 10 carbon atoms inclusive are cyclopropyl, cyclobutyl, cyclopentyl, cyclohexyl, cycloheptyl, cyclooctyl, cyclononyl, and cyclodecyl.

Examples of bicycloalkyl of from 3 to 10 carbon atoms, inclusive include norbornyl.

Examples of polycycloalkyl from 3 to 10 carbon atoms, inclusive, include adamantyl.

The utility of the instant compounds and their inhibition of the formation of serum cholesterol can be demonstrated via the following standarized test procedure:

Male Charles River CD rats initially weighing 180—250 g apiece are randomized in groups of 6, housed in a reverse light cycle (12:12) room, and maintained therein on a standard rat diet plus water *ad libitum*.

To each animal in a group, after at least 3, but not more than 6 days, 5 mg/kg of 20,25-diazachlolesterol dissolved in 0.2 ml of physiological saline containing 0.1 percent of polyoxyethylene sorbitan monooleate (Tween 80) is intragastrically administered on each of 7 consecutive days, during the last 4 of which the test compound is concurrently and identically administered at a pre-selected, daily dose (commonly 5 mg/kg intragastrically). Controls are provided by a second group of animals identically treated except that test compound is omitted. Within 2—4 hr after treatment is completed, and 5—7 hrs. into the dark cycle, the animals are anesthetized with diethyl ether and thereupon killed. Livers are quickly removed, washed with a chilled homogenization medium (preparable by dissolving 102.7 g of sucrose, 3.8 g of sodium edetate, and 0.8 g of diethiothreitol in water *q.s.* 1000 ml), blotted dry, weighed, and homogenized (using 2 ml of the aforesaid chilled medium for each g of liver). The homogenates are centrifuged at 4°C and 15,000×g for 15 min., whereupon the supernatants are separated and centrifuged at 4°C and 100,000×g for 60 min. The resultant supernatants are discarded and the residues suspended in half the volume of homogenization medium previously employed (i.e., 1 ml for each gram of residue). HMG CoA reductase activity is assayed substantially in accordance with procedures described by L. W. White *et al.* in Biochemistry, *9*, 2713 (1970), M. S. Brown *et al.* in Biochim. Biophys. Acta, *409*, 39 (1975).

Protein is determined by the method of O. H. Lowry *et al.*, J. Biol. Chem., *193*, 265 (1951). The data obtained is converted to specific activity (mmol/20 min./mg protein) for each animal, from which group mean(n) and per cent change, relative to controls, are calculated. A statistically significant response ($p \leq 0.05$) is the criterion for HMG CoA reductase inhibition/stimulation.

By virtue of this activity the compounds of formula I are useful in treating type 2 hypercholesterolemia

(TTH-2) in humans and animals. A physician or veterinarian of ordinary skill could readily determine a subject who has TTH-2 symptoms. Regardless of the route of administration selected, the compounds of the present invention are formulated into pharmaceutically acceptable dosage forms by conventional methods known to the pharmaceutical art.

The compounds can be administered in such oral unit dosage forms such as tablets, capsules, pills, powders, or granules. They also may be administered rectally, vaginally in such forms as suppositories, interparenterally, subcutaneously, or intramuscularly, using forms kown to the pharmaceutical art. In general, the preferred form of administration is orally.

An effective but non-toxic quantity of the compound is employed in treatment. The dosage regimen for preventing or treating TTH-2 by the compounds of this invention is selected in accordance with a variety of factors including the type, age, weight, sex, and medical condition of the mammal, the severity of the TTH-2, the route of administration and the particular compound employed. An ordinarily skilled physician or veterinarian will readily determine and prescribe the effective amount of the Anti-TTH-2 agent to prevent or arrest the progress of the condition. In so preceeding, the physician or veterinarian could employ relatively low dosages at first, subsequently increasing the dose until a maximum response is obtained.

Initial dosages of the compounds of the invention are ordinarily in the area of 10 mg/kg up to 200 mg/kg orally. When other forms of administration are employed equivalent doses are administered.

The general procedure for producing the compounds of the instant invention is outlined on Chart A. It is similar to the general procedure used to produce the compounds of U.S. Patent 3,818,080. The carboxylic esters used as starting materials, which are not readily available from commercial sources or by esterification of available acids, can be prepared by for example the routes outlined on Chart B.

Description of the preferred embodiments

The operation of this invention is further elaborated by the representative examples below:

Example 1

11-(Triphenylphosphonium)undecanoic acid bromide

A mixture of 137 g of 11-bromoundecanoic acid and 137 g of triphenylphosphine is stirred for two days under nitrogen in 2 l of dry refluxing toluene. Overnight cooling, ca 0°, gives oily crystals from which toluene is removed by decanting. Washing with ether gives ca. 275 g of crude product. Recrystallization by precipitation from dichloromethane using diethylether produces a white powder, m.p. 86—90°C. Structure assignment of the product compound is supported by elemental analysis and by infrared and nmr spectroscopy.

Example 2

11-(Cycloheptylidene)undecanoic acid

To 10.9 g (0.23 mole) of 50 percent sodium hydride in mineral oil is added, under a nitrogen atmosphere, 200 ml of dry dimethylsulfoxide, and the mixture is heated at 60°C until gas evolution ceases within *ca.* 1 hr. The mixture is cooled to 20 to 25° and a solution of 54.5 g (0.10 mole) of 11-(triphenylphosphonium)undecanoic acid bromide in 200 ml of dimethylsulfoxide is added dropwise at less than 25°. After addition is completed, the mixture is cooled to 0° to 5°C and diluted with 300 ml of dry tetrahydrofuran. A solution of 16.2 g (0.14 mole) of cycloheptanone in 100 ml of tetrahydrofuran is added dropwise with stirring to the above mixture, the temperature being kept at less than 5°. After addition is completed, the stirred reaction mixture is allowed to warm to room temperature for 12 to 24 hrs. The mixture is then diluted with water and acidified with dilute sulfuric acid, and the product is extracted using two portions of Skellysolve B. When the organic layer is washed thoroughly with four portions of dilute sulfuric acid, an oily material containing triphenylphosphine oxide separates and is discarded. The remaining organic layer is dried with anhydrous sodium sulfate, concentrated to an oil and dissolved in 50 ml of warm Skellysolve A. The white crystalline (10-carboxydecyl)diphenylphosphine oxide which precipitates upon cooling to 0°C is collected and discarded. The solution in Skellysolve A is diluted to one l with additional Skellysolve A, decolorized with charcoal and filtered. The solvent is evaporated and the residue dried *in vacuo* to give 16.2 g of product as a homogeneous, pale yellow oil. The compound is used for subsequent reactions without further purification.

Example 3

Methyl 11-(cycloheptylidene)undecanoate

The methyl ester of 11-(cycloheptylidene)undecanoic acid is prepared by dissolving 16.2 g of the carboxylic acid in 300 ml of methanol to which is then added 1 ml of thionyl chloride. After three hours at room temperature, the solution is concentrated *in vacuo*, dissolved in Skellysolve A, and washed with dilute sodium bicarbonate. After residual solids are removed by filtration, the solution is washed with water, dried, decolorized with charcoal and filtered, and concentrated by evaporation of solvent. The oily residue is distilled at reduced pressure. Fractions with a boiling range of 138 to 144°C at 0.15 mm Hg afford 12.4 g of product as a colorless oil. Structure assignment is supported by elemental analysis and by infrared and nmr spectroscopy.

Example 4

Methyl 11-(cycloheptyl)undecanoate

Hydrogenation of methyl 11-(cycloheptylidene)undecanoate (12.4 g) is effected in tetrahydrofuran at room temperature under 2 p.s.i. hydrogen using 5 percent palladium on charcoal as catalyst. Upon removal of insolubles by filtration, the mixture is concentrated *in vacuo*. The residue is dissolved in Skellysolve A, decolorized over charcoal and filtered. The solvent is evaporated and the residue is distilled at reduced pressure. Those fractions having a boiling range of 158 to 160°C at 0.5 mm Hg pressure afford 9.2 g of product as a colorless oil. Structure assignement is supported by elemental analysis and by infrared and nmr spectroscopy.

Example 5

Methyl 11-(cyclohexyl)undecanoate

Hydrogenation of 11-phenylundecanoic acid (50.0 g) is effected in 200 ml of methanol at 60° under 60 p.s.i. hydrogen using 5 percent rhodium on charcoal as catalyst. After removing catalyst by filtration, the filtrate (now 400 ml) is diluted with 800 ml of dichloromethane. Esterification of the intermediate cyclohexylundecanoic acid is effected by acidifying the solution with 1 ml of sulfuric acid and reluxing for *ca*. 6 hours under magnesium sulfate in a Soxhlet extractor. Excess potassium carbonate is added to the cooled solution, which is then filtered. The filtrate is washed with saturated sodium chloride and 10 percent sodium bicarbonate, and the organic phase is dried over sodium sulfate and refiltered. The solution is concentrated *in vacuo*, and the residue is dissolved in Skellysolve A. After a small residue is removed by filtration, the solution is again concentrated to an oil which is purified by distillation under reduced pressure. A large fraction boiling at 137—141°C at 0.3 mm Hg pressure affords 49.1 g of the product as a homogeneous colorless oil. Structure assignment is supported by elemental analysis and by infrared and nmr spectroscopy.

Example 6

1,1-Bis(allyl)-5-phenylpentanol

To an excess of magnesium metal (6.2 g, 0.26 mole) stirred in 250 ml of dry tetrahydrofuran under a nitrogen atmosphere is added 2 ml each of allyl bromide and allyl magnesium bromide (1M in diethyl ether) and a catalytic amount of iodine. After the reaction begins, a solution of 21.6 g (0.11 mole) of methyl 5-phenylpentanoate and 31.3 g (0.26 mole) of allyl bromide in 50 ml of dry tetrahydrofuran is added dropwise to the reaction vessel. (Methyl 5-phenylpentanoate is prepared from the corresponding carboxylic acid according to the method of Example 3, except that diethyl ether replaces Skellysolve A and the product distills at 80 to 86°C at 0.44 mm Hg.) The reaction mixture is heated at reflux for *ca*. 1 hour, after which the reaction is quenched with methanol. The mixture is diluted with diethyl ether and washed with saturated aqueous ammonium chloride. The solution is dried, filtered, and concentrated *in vacuo* to an oil. Drying the oil at reduced pressure (2 mm Hg, 40°) gives 28.1 g of the carbinol product. Structure assignment is supported by infrared and nmr spectroscopy.

Example 7

3-Hydroxy-3-[10-(cyclohexyl)decyl]glutaric acid

The bis-allyl carbinol (6.12 g), prepared from methyl 11-(cyclohexyl)undecanoate [See Example 5] by the method of Example 6, is dissolved in a mixture of 100 ml each of dichloromethane and ethyl acetate and is cooled to *ca*. −60 to −30°C. Ozone is bubbled into the solution until a blue color persists, *ca*. 1 hour. The solution is purged with oxygen ($O_2$) and then added dropwise, with stirring, to 60 ml of cold acetic acid. The solution is heated gently to distill off dichloromethane and then is allowed to cool. A solution of 10 ml of water, 20 ml of 10 percent sulfuric acid, 20 ml of 30 percent hydrogen peroxide, and 30 ml of acetic acid is added dropwise. The reaction mixture is heated to *ca*. 85°C to boil off some of the solvent and then heated at reflux for *ca*. 2 hours. The solution is concentrated *in vacuo* and then diluted with 800 ml of water, preducing an oily precipitate. The crude product is extracted into diethyl ether, which is washed three times with dilute sulfuric acid, then once with dilute sulfuric acid containing 20 percent sodium hydrogen sulfite, and again with dilute sulfuric acid, and then extracted twice into dilute sodium hyroxide. (The organic phase is dried, filtered, and concentrated to yield less than 1 g of an oil which is discarded). The sodium hydroxide extracts are combined and acidified, and the crude product is extracted into diethyl ether. After washing, the ether phase is dried and concentrated to an oil which is purified by column chromatography. Appropriate fractions are concentrated and dried at 20°C under reduced pressure (0.5 mm Hg), giving 1.94 g of the dicarboxylic acid product as a yellow glass. Structure assignment is supported by elemental analysis and by infrared and nmr spectroscopy.

Example 8

3-Hydroxy-3-[4-(cyclohexyl)butyl]glutaric acid

The dicarboxylic acid product is prepared by the method of Example 7 from 33.7 g of the corresponding bis-allyl carbinol, which in turn is prepared by the methods of Examples 5 and 6 from 5-phenylpentanoic acid. Instead of chromatography, crystallization is used to purify the product. A solution of product in 150 ml of acetone is diluted with 700 ml of Skellysolve B and concentrated until cloudy (*ca*. 300

ml). Cooling at 0° gives 20.9 g of product as a white crystalline powder. Structure assignment is supported by elemental analysis and by infrared and nmr spectroscopy.

Example 9

3-Hydroxy-3-[6-(cyclohexyl)hexyl]glutaric acid

The dicarboxylic acid product is prepared by the method of Example 7 from 6.2 g of the corresponding bis-allyl carbinol, which in turn is prepared by the methods of Examples 5 and 6 from 7-phenylheptanoic acid. Instead of chromatography, crystallization from 20 ml of cold diethyl ether is used to purify the product, isolated as 1.5 g of white crystalline powder. Structure assignment is supported by elemental analysis and by infrared and nmr spectroscopy.

Example 10

3-Hydroxy-3-[8(cyclohexyl)octyl]glutaric acid

The dicarboxylic acid product is prepared by the method of Example 7 from 4.9 g of the corresponding bis-allyl carbinol, which in turn is prepared by the methods of Examples 5 and 6 from 9-phenylnonanoic acid. Instead of chromatography, crystallization from 75 ml of 30 percent diethyl ether in Skellysolve B is used to purify the product, isolated as 2.5 g of white crystalline powder. Structure assignment is supported by elemental analysis and by infrared and nmr spectroscopy.

Example 11

3-Hydroxy-3-[11-(cyclohexyl)undecyl]glutaric acid

The dicarboxylic acid product is prepared by the method of Example 7 from the corresponding bis-allyl carbinol, which in turn is prepared by the methods of Examples 2, 3, 4, and 6 from benzaldehyde and 11-(triphenylphosphonium) undecanoic acid bromide. The product is isolated by crystallization as 3.3 g of a white solid. Structure assignment is supported by elemental analysis and by infrared and nmr spectroscopy.

Example 12

3-Hydroxy-3-[13-(cyclohexyl)tridecyl]glutaric acid

The dicarboxylic acid is prepared by the method of Example 7 from the corresponding bis-allyl carbinol, which in turn is prepared by the methods of Examples 2, 3, 5, and 6 from 19.2 g of cinnamaldehyde and 54.5 g of 11-(triphenylphosphonium)undecanoic acid bromide. (The cinnamyl double bond is reduced during this process). The product is isolated by crystallization as 16.0 g of a white powder. Structure analysis is supported by elemental analysis and by infrared and nmr spectroscopy.

Example 13

3-Hydroxy-3-[11-(cyclohexyl)dodecyl]glutaric acid

The dicarboxylic acid product is prepared by the method of Example 7 from the corresponding bis-allyl carbinol, which in turn is prepared by the methods of Examples 2, 3, 4, and 6 from 17.4 g of acetophenone and 54.5 g of 11-(triphenylphosphonium)undecanoic acid bromide. The product is isolated by crystallization as 8.9 g of a white solid. Structure assignment is supported by elemental analysis and by infrared and nmr spectroscopy.

Example 14

3-Hydroxy-3-[11-(cyclohexyl)pentadecyl]glutaric acid

The dicarboxylic acid product is prepared by the method of Example 7 from the corresponding bis-allyl carbinol, which in turn is prepared by the methods of Examples 2, 3, 4, and 6 from 23.5 g of phenyl butyl ketone and 54.5 g of 11-(triphenylphosphonium)undecanoic acid bromide. The product is isolated by chromatography as 8.2 g of a colorless oil. Structure assignment is supported by elemental analysis and by infrared and nmr spectroscopy.

Example 15

3-Hydroxy-3-[11,11-(biscyclohexyl)undecyl]glutaric acid

The dicarboxylic acid product is prepared by the method of Example 7 from the corresponding bis-allyl carbinol, which in turn is prepared by the methods of Examples 2, 3, 4, and 6 from 18.2 g of benzophenone and 54.5 g of 11-(triphenylphosphonium)undecanoic acid bromide. The product is isolated by crystallization as 2.6 g of a white solid. Structure assignment is supported by elemental analysis and by infrared and nmr spectroscopy.

Example 16

3-Hydroxy-3-[6-(4-tert-butylcyclohexyl)hexyl]glutaric acid

The dicarboxylic acid is prepared by the method of Example 7 from the corresponding bis-allyl carbinol, which in turn is prepared by the methods of Examples 2, 3, 4, and 6 from 8.6 g of 4-tert-butyl cyclohexanone and 18 g of 6-(triphenylphosphonium)heptanoic acid bromide. The product is isolated by crystallization as 3.3 g of a white powder. Structure assignment is supported by elemental analysis and by infrared and nmr spectroscopy.

Example 17
3-Hydroxy-3-[10-(4-tert-butylcyclohexyl)decyl]glutaric acid

The dicarboxylic acid is prepared by the method of Example 7 from the corresponding bis-allyl carbinol, which in turn is prepared by the methods of Examples 2, 3, 4 and 6 from 22.4 g of 4-tert-butylcyclohexanone and 54.5 g of 10-(triphenylphosphonium)undecanoic acid bromide. The product is isolated by crystallization as 16.2 g of a white powder. Structure analysis is supported by elemental assignment and by infrared and nmr spectroscopy.

Example 18
3-Hydroxy-3-[10-(1-methylcyclohexyl)decyl]glutaric acid

The dicarboxylic acid is prepared by the method of Example 7 from the corresponding bis-allyl carbinol, which in turn is prepared by the methods of Examples 2, 3, 4 and 6 from 8.7 g of (1-methylcyclohexyl)carboxaldehyde and 24. g of 10-(triphenylphosphonium)decanoic acid bromide. The product is isolated by chromotography as of a light yellow glass. Structure assignment is supported by elemental analysis and by infrared and nmr spectroscopy.

Example 19
3-Hydroxy-3-[11-(1-methylcyclohexyl)undecyl]glutaric acid

The dicarboxylic acid is prepared by the method of Example 7 from the corresponding bis-allyl carbinol, which in turn is prepared by the methods of Examples 2, 3, 4, and 6 from 8.7 g of (1-methylcyclohexyl)carboxaldehyde and 25. g of 11-(triphenylphosphonium)undecanoic acid bromide. The product is isolated by chromatography as a light yellow glass. Structure assignment is supported by elemental analysis and by infrared and nmr spectroscopy.

Example 20
3-Hydroxy-3-[10-(cycloheptyl)decyl]glutaric acid

The dicarboxylic acid product is prepared by the method of Example 7 from the corresponding bis-allyl carbinol, which in turn is prepared by the method of Examples 2, 3, 4, and 6 from 15.7 of cycloheptanone. The product is isolated by crystallization as 6.4 g of a white solid. Structure assignment is supported by elemental analysis and by infrared and nmr spectroscopy.

Example 21
3-Hydroxy-3-(11-ethyltridecyl)glutaric acid

The dicarboxylic acid product is prepared by the method of Example 7 from the corresponding bis-allyl carbinol, which in turn is prepared by the methods of Examples 2, 3, 4, and 6 from 12.5 g of 3-pentanone and 54.5 g of 11-(triphenylphosphonium)undecanoic acid bromide. The product is isolated by crystallization as 9.4 g of a white solid. Structure assignment is supported by elemental analysis and by infrared and nmr spectroscopy.

Example 22
3-Hydroxy-3-(10,10-dimethylundecyl)glutaric acid

The dicarboxylic acid is prepared by the method of Example 7 from the corresponding bis-allyl carbinol, which in turn is prepared by the methods of Examples 2, 3, 4, and 6 from 3.0 g of pivalaldehyde and 11.6 g of 9-(triphenylphosphonium)nonanoic acid bromide. The product is isolated by crystallization as 1.24 g of a white powder. Structure assignment is supported by elemental analysis and by infrared and nmr spectroscopy.

Example 23
3-Hydroxy-3-(11,11-dimethyldodecyl)glutaric acid

The dicarboxylic acid is prepared by the method of Example 7 from the corresponding bis-allyl carbinol, which in turn is prepared by the methods of Examples 2, 3, 4, and 6 from 5.7 g of pivalaldehyde and 24.4 g of 10-(triphenylphosphonium)decanoic acid bromide. The product is isolated by crystallization as 7.4 g of a white powder. Structure assignment is supported by elemental analysis and by infrared and nmr spectroscopy.

Example 24
3-Hydroxy-3-(12,12-dimethyltridecyl)glutaric acid

The dicarboxylic acid product is prepared by the method of Example 7 from the corresponding bis-allyl carbinol, which in turn is prepared by the methods of Examples 2, 3, 4, and 6 from 12.5 g of pivalaldehyde and 54.5 g of 11-(triphenylphosphonium)undecanoic acid bromide. The product is isolated by crystallization from diethylether/Skellysolve A as 11.8 g of a white solid. Structure assignment is supported by elemental analysis and by infrared and nmr spectroscopy.

Example 25

3-Hydroxy-3-[11-(2-norbornyl)undecyl]glutaric acid

The dicarboxylic acid is prepared by the method of Example 7 from the corresponding bis-allyl carbinol, which in turn is prepared by the methods of Examples 2, 3, 4, and 6 from 17.4 g of 5-norbornene-2-carboxaldehyde and 54.5 g of 11-(triphenylphosphonium)undecanoic acid bromide. (The norbornene double bond is reduced during this process.) The product is isolated by crystallization as 16.9 g of a white powder. Structure assignment is supported by elemental analysis and by infrared and nmr spectroscopy.

Example 26

3-Hydroxy-3-[10-(2-adamantyl)decyl]glutaric acid

The dicarboxylic acid is prepared by the method of Example 7 from the corresponding bis-allyl carbinol, which in turn is prepared by the methods of Examples 2, 3, 4, and 6 from 21.8 g of 2-adamantanone and 54.5 g of 11-(triphenylphosphonium)undecanoic acid bromide, except that the intermediate methyl-11-(2-adamantyl)undecanoate was purified by chromatography before reacting in the Grignard reaction. The product is isolated by crystallization as 5.8 g of a white powder. Structure assignment is supported by elemental analysis and by infrared and nmr spectroscopy.

Example 27

3-Hydroxy-3-[11-(1-adamantyl)undecyl]glutaric acid

The dicarboxylic acid is prepared by the method of Example 7 from the corresponding bis-allyl carbinol, which in turn is prepared by the methods of Examples 2, 3, 4, and 6 from 23.8 g of 1-adamantylcarboxaldehyde and 54.5 g of 11-(triphenylphosphonium)undecanoic acid bromide. The product is isolated by chromotography as a light yellow glass. Structure assignment is supported by elemental analysis and by infrared and nmr spectroscopy.

Example 28

3-Hydroxy-3-[4-(decyl)cyclohexyl]glutaric acid

The dicarboxylic acid product is prepared by the method of Example 7 from 22.0 g of the corresponding bis-allyl carbinol, which in turn is prepared by the method of Example 6 from methyl 1-[4-(decyl)cyclohexyl]carboxylate. Recrystallization from 50 ml of Skellysolve B is used to purify the product, isolated as 9.7 g of white crystalline powder. Structure assignment is supported by elemental analysis and by infrared and nmr spectroscopy.

Example 29

3-Hydroxy-3-[10-(cyclohexyloxy)decyl]glutaric acid and 3-hydroxy-3-(10-hydroxydecyl)glutaric acid

Reduction of 23.5 g of methyl 11-phenoxyundecanoate is effected in 240 ml of methanol at 60°C under 60 p.s.i. of hydrogen using 5 percent rhodium on charcoal. After hydrogen uptake is complete and catalyst is removed by filtration, the filtrate is concentrated and the residue is distilled. Methyl 11-(cyclohexyloxy)undecanoate (21.0 g) is collected as a colorless oil (boiling range 140 to 142°C at 0.1 mm Hg) having expected elemental analysis and infrared and nmr spectra. The ester is converted to the appropriate bis-allyl carbinol by the method of Example 6. Chromatography of the crude intermediate product affords 11.6 g of the bis-allyl carbinol as an oil, which is then ozonized according to the method of Example 7. Two major components are separated by column chromatography. Eluate fractions containing 3-hydroxy-3-[10-(cyclohexyloxy)decyl]glutaric acid produce crystals which are recrystallized from diethyl ether-Skellysolve A, giving 1.4 g of white crystalline product. Later eluate fractions containing 3-hydroxy-3-(10-hydroxydecyl)glutaric acid are similarly recrystallized, giving less than 2 g of oily white solid. Structure assignments of both produts are supported by elemental analyses and by infrared and nmr spectroscopy.

Example 30

3-Hydroxy-3-[10-(cyclohexyl)decyl]glutaric acid, monomethyl ester

To 7.0 g (18.9 mmole) of 3-hydroxy-3-[10-(cyclohexyl)decyl]glutaric acid [from Example 7] dissolved in 100 ml of tetrahydrofuran is added 4.0 g (19.5 mmole) of dicyclohexylcarbodiimide. After one hour at room temperature the mixture is filtered to remove dicyclohexylurea, and the filtrate is concentrated *in vacuo*. The residue is dissolved in 250 ml of methanol containing two drops of pyridine. After reaction is complete, the mixture is concentrated to dryness. The crude product is taken up in Skellysolve A, and then filtered to remove additional dicyclohexylurea. Concentration of filtrate to dryness and drying at 1 mm Hg (40°) affords 7.4 g of the product as a pale yellow oil. Structure assignment is supported by elemental analysis and by infrared and nmr spectroscopy.

CHART A

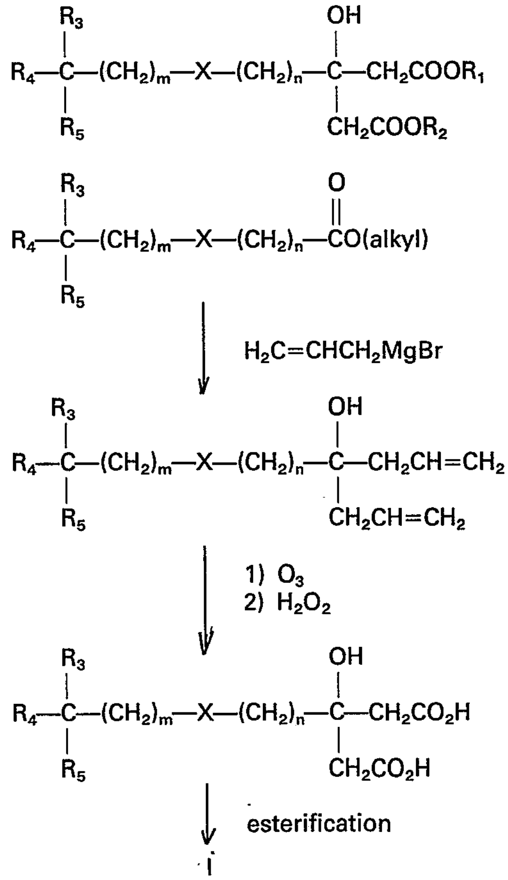


I

II

III

IV

CHART B

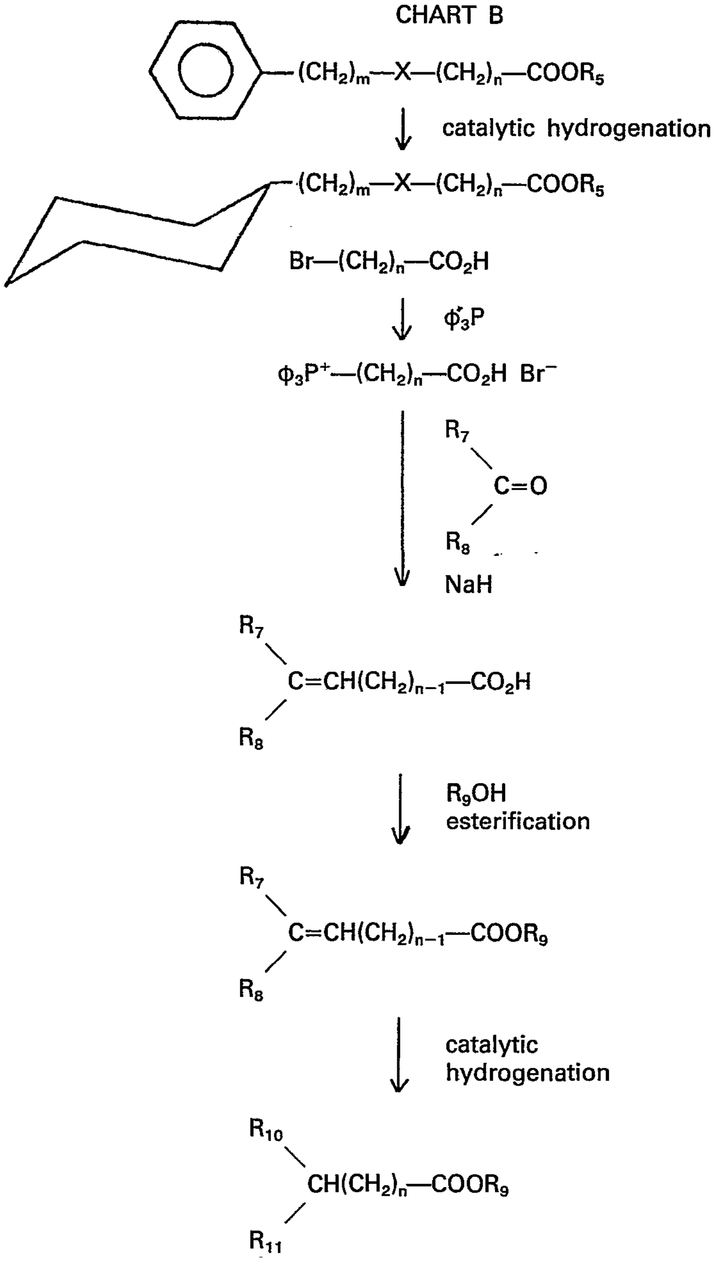


XI

XII

## Claims

1. A compound of the formula:

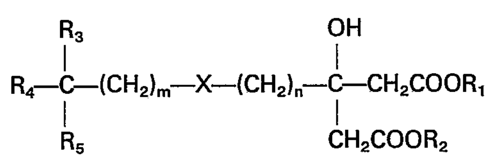

wherein

$R_1$ and $R_2$ are:

a) hydrogen; or

b) alkyl of from 1 to 6 carbon atoms, inclusive;

$R_1$ and $R_2$ each being the same or different;

wherein

$R_3$, $R_4$ and $R_5$ are:

a) hydrogen;

b) alkyl of from 1 to 6 carbon atoms, inclusive; or

c) cycloalkyl, bicycloalkyl, or polycycloalkyl of from 3 to 10 carbon atoms, inclusive;

$R_3$, $R_4$ and $R_5$, each being the same or different or wherein any two or all three of $R_3$, $R_4$ and $R_5$ are taken

together to form a saturated hydrocarbon ring having from 3 to 10 carbon atoms, inclusive;
wherein
m is an integer of from zero to 15, inclusive;
wherein
n is an integer of from zero to 15, inclusive;
wherein
X is:
a) $-CH_2-$; or
b) branched alkylene of from 2 to 15 carbon atoms; or
c) cycloalkylene, bicycloalkylene, or polycycloalkylene of from 4 to 10 carbon atoms; or
d) —O—
with the exception of those compounds of the formula wherein $R_1$ and $R_2$ are both hydrogen and the radical

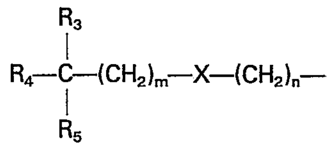

is ethyl, n-propyl isopropyl, n-butyl, t-butyl, isobutyl, n-decyl, pentadecyl or cyclopentylmethyl, and with the exception of the dimethylesters of 3-hydroxy-3-propylglutaric acid and 3-hydroxy-3-isopropylglutaric acid.

2. A compound according to Claim 1 wherein X is $-CH_2-$ with the exception of those comopunds of the formula wherein $R_1$ and $R_2$ are both hydrogen and the radical

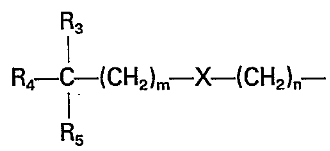

is ethyl, n-propyl n-butyl, n-decyl, pentadecyl or cyclopentylmethyl and with the exception of the dimethyl ester of 3-hydroxy-3-propylglutaric acid.

3. 3-Hydroxy-3-[10-(cyclohexyl)decyl]glutaric acid, a compound according to Claim 2.
4. 3-Hydroxy-3-[4-(cyclohexyl)butyl]glutaric acid, a compound according to Claim 2.
5. 3-Hydroxy-3-[6-(cyclohexyl)hexyl]glutaric acid, a compound according to Claim 2.
6. 3-Hydroxy-3-[8-(cyclohexyl)octyl]glutaric acid, a compound according to Claim 2.
7. 3-Hydroxy-3-[11-(cyclohexyl)undecyl]glutaric acid, a compound according to Claim 2.
8. 3-Hydroxy-3-[13-(cyclohexyl)tridecyl]glutaric acid, a compound according to Claim 2.
9. 3-Hydroxy-3-[11,11-(dicyclohexyl)undecyl]glutaric acid, a compound according to Claim 2.
10. 3-Hydroxy-3-[10-(cycloheptyl)decyl]glutaric acid, a compound according to Claim 2.
11. 3-Hydroxy-3-[11-(cyclohexyl)pentadecyl]glutaric acid, a compound according to Claim 2.
12. 3-Hydroxy-3-[11-(cyclohexyl)dodecyl]glutaric acid, a compound according to Claim 2.
13. 3-Hydroxy-3-[10-(cyclohexyl)decyl]glutaric acid, monomethylester, a compound according to Claim 2.
14. 3-Hydroxy-3-[10-(1-methylcyclohexyl)decyl]glutaric acid, a compound according to Claim 2.
15. 3-Hydroxy-3-[11-(1-methylcyclohexyl)undecyl]glutaric acid, a compound according to Claim 2.
16. 3-Hydroxy-3-[11-(2-norbornyl)undecyl]glutaric acid, a compound according to Claim 2.
17. 3-Hydroxy-3-[10-(2-adamantyl)decyl]glutaric acid, a compound according to Claim 2.
18. 3-Hydroxy-3-[11-(1-adamantyl)undecyl]glutaric acid, a compound according to Claim 2.
19. 3-Hydroxy-3-(11-ethyltridecyl)glutaric acid, a compound according to Claim 2.
20. 3-Hydroxy-3-(10,10-dimethylundecyl)glutaric acid, a compound according to Claim 2.
21. 3-Hydroxy-3-(11,11-dimethyldodecyl)glutaric acid, a compound according to Claim 2.
22. 3-Hydroxy-3-(12,12-dimethyltridecyl)glutaric acid, a compound according to Claim 2.
23. A compound according to Claim 1 wherein X is cycloalkylene.
24. 3-Hydroxy-3-[6-(4-*tert*-butylcyclohexyl)hexyl]glutaric acid, a compound according to Claim 23.
25. 3-Hydroxy-3-[10-(4-*tert*-butylcyclohexyl)decyl]glutaric acid, a compound according to Claim 23.
26. 3-Hydroxy-3-[4-(decyl)cyclohexyl]glutaric acid, a compound according to Claim 23.
27. A compound according to Claim 1 wherein X is —O—.
28. 3-Hydroxy-3-[10-(cyclohexyloxy)decyl]glutaric acid, a compound according to Claim 27.
29. 3-Hydroxy-3-(10-hydroxydecyl)glutaric acid, a compound according to Claim 27.

**Patentansprüche**

1. Verbindung der Formel

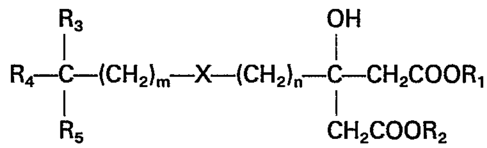

worin
$R_1$ und $R_2$ sind:
a) Wasserstoff; oder
b) Alkylgruppen mit 1 bis 6 Kohlenstoffatomen einschließlich;
wobei
$R_1$ und $R_2$ jeweils gleich oder verschieden sind;
worin
$R_3$, $R_4$ und $R_5$ sind:
a) Wasserstoff;
b) Alkylgruppen mit 1 bis 6 Kohlenstoffatomen einschließlich; oder
c) Cycloalkyl-, Bicycloalkyl- oder Polycycloalkylgruppen mit 3 bis 10 Kohlenstoffatomen einschließlich;
wobei
$R_3$, $R_4$ und $R_5$ jeweils gleich oder verschieden sind, oder worin beliebige zwei oder alle drei der Reste $R_3$, $R_4$ und $R_5$ zusammengenommen einen gesättigten Kohlenwasserstoffring mit 3 bis 10 Kohlenstoffatomen einschießlich bilden;
worin
m eine ganze Zahl von 0 bis 15 einschließlich ist;
worin
n eine ganze Zahl von 0 bis 15 einschließlich ist;
worin X ist:
a) $-CH_2-$; oder
b) eine verzweigte Alkylengruppe mit 2 bis 15 Kohlenstoffatomen; oder
c) eine Cycloalkylen-, Bicycloalkylen- oder Polycycloalkylengruppe mit 4 bis 10 Kohlenstoffatomen; oder
d) $-O-$
mit der Ausnahme derjenigen Verbindungen der Formal, worin $R_1$ und $R_2$ beide Wasserstoff bedeuten und der Rest

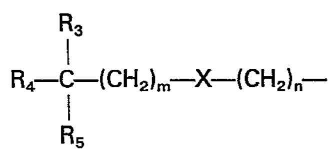

den Ethyl-, n-Propyl-, Isopropyl-, n-Butyl-, t-Butyl-, Isobutyl-, n-Decyl-, Pendadecyl- oder Cyclopentylmethylrest bedeutet, und mit Ausnahme der Dimethylester von 3-Hydroxy-3-propylglutarsäure und 3-Hydroxy-3-isopropylglutarsäure.

2. Verbindung gemäß Anspruch 1, worin X $-CH_2-$ bedeutet, mit Ausnahme derjenigen Verbindungen der Formel, worin $R_1$ und $R_2$ beide Wasserstoff bedeuten und der Rest

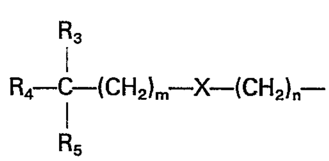

den Ethyl-, n-Propyl-, n-Butyl-, n-Decyl-, Pentadecyl- oder Cyclopentylmethylrest bedeutet, und mit Ausnahme der Dimethylesters von 3-Hydroxy-3-propylglutarsäure.

3. 3-Hydroxy-3-[10(cyclohexyl)decyl]-glutarsäure, eine Verbindung gemäß Anspruch 2.
4. 3-Hydroxy-3-[4-(cyclohexyl)butyl]-glutarsäure, eine Verbindung gemäß Anspruch 2.
5. 3-Hydroxy-3-[6-(cyclohexyl)hexyl]-glutarsäure, eine Verbindung gemäß Anspruch 2.
6. 3-Hydroxy-3-[8-(cyclohexyl)octyl]-glutarsäure, eine Verbindung gemäß Anspruch 2.
7. 3-Hydroxy-3-[11-(cyclohexyl)undecyl]-glutarsäure, eine Verbindung gemäß Anspruch 2.
8. 3-Hydroxy-3-[13-(cyclohexyl)tridecyl]-glutarsäure, eine Verbindung gemäß Anspruch 2.
9. 3-Hydroxy-3-[11,11-(dicyclohexyl)undecyl]-glutarsäure, eine Verbindung gemäß Anspruch 2.

10. 3-Hydroxy-3-[10-(cycloheptyl)decyl]-glutarsäure, eine Verbindung gemäß Anspruch 2.

11. 3-Hydroxy-3-[11-(cyclohexyl)pentadecyl]-glutarsäure, eine Verbindung gemäß Anspruch 2.

12. 3-Hydroxy-3-[11-(cyclohexyl)dodecyl]-glutarsäure, eine Verbindung gemäß Anspruch 2.

13. 3-Hydroxy-3-[10-(cyclohexyl)decyl]-glutarsäure-monomethylester, eine Verbindung gemäß Anspruch 2.

14. 3-Hydroxy-3-[10-(1-methylcyclohexyl)decyl]-glutarsäure, eine Verbindung gemäß Anspruch 2.

15. 3-Hydroxy-3-[11-(1-methylcyclohexyl)undecyl]-glutarsäure, eine Verbindung gemäß Anspruch 2.

16. 3-Hydroxy-3-[11-(2-norbornyl)undecyl]-glutarsäure, eine Verbindung gemäß Anspruch 2.

17. 3-Hydroxy-3-[10-(2-adamantyl)decyl]-glutarsäure, eine Verbindung gemäß Anspruch 2.

18. 3-Hydroxy-3-[11-(1-adamantyl)undecyl]-glutarsäure, eine Verbindung gemäß Anspruch 2.

19. 3-Hydroxy-3-(11-ethyltridecyl)-glutarsäure, eine Verbindung gemäß Anspruch 2.

20. 3-Hydroxy-3-(10,10-dimethylundecyl)-glutarsäure, eine Verbindung gemäß Anspruch 2.

21. 3-Hydroxy-3-(11,11-dimethyldodecyl)-glutarsäure, eine Verbindung gemäß Anspruch 2.

22. 3-Hydroxy-3-(12,12-dimethyltridecyl)-glutarsäure, eine Verbindung gemäß Anspruch 2.

23. Verbindung gemäß Anspruch 1, worin X eine Cycloalkylengruppe ist.

24. 3-Hydroxy-3-[6-(4-tert.-butylcyclohexyl)hexyl]-glutarsäure, eine Verbindung gemäß Anspruch 23.

25. 3-Hydroxy-3-[10-(4-tert.-butylcyclohexyl)decyl]-glutarsäure, eine Verbindung gemäß Anspruch 23.

26. 3-Hydroxy-3-[4-(decyl)cyclohexyl]-glutarsäure, eine Verbindung gemäß Anspruch 23.

27. Verbindung gemäß Anspruch 1, worin X —O— ist.

28. 3-Hydroxy-3-[10-(cyclohexyloxy)decyl]-glutarsäure, eine Verbingung gemäß Anspruch 27.

29. 3-Hydroxy-3-(10-hydroxydecyl)-glutarsäure, eine Verbindung gemäß Anspruch 27.

**Revendications**

1. Composé de formule:

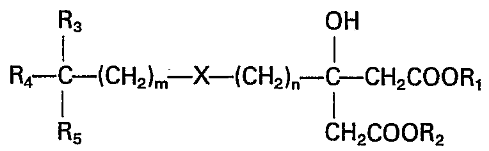

dans laquelle

$R_1$ et $R_2$ représentent:

a) un atome d'hydrogène; ou

b) un groupe alkyle ayant 1 à 6 atomes de carbone inclusivement;

$R_1$ et $R_2$ étant identiques ou différents;

$R_3$, $R_4$ et $R_5$ représentent:

a) un atome d'hydrogène;

b) un groupe alkyle ayant 1 à 6 atomes de carbone inclusivement;

c) un groupe cycloalkyle, bicycloalkyle ou polycycloalkyle ayant 3 à 10 atomes de carbone, inclusivement;

$R_3$, $R_4$ et $R_5$ représentent:

a) un atome d'hydrogène

b) un groupe alkyle ayant 1 à 6 atomes de carbone, inclusivement 2 ou

c) un groupe cycloalkyle, bicycloalkyle ou polycycloalkyle ayant 3 à 10 atomes de carbone, inclusivement;

$R_3$, $R_4$ et $R_5$, étant identiques ou différents, et 2 quelconques des symboles $R_3$, $R_4$ et $R_5$, ou l'ensemble de ces 3 symboles formant, quand ils sont pris ensemble, un noyau hydrocarboné saturé ayant de 3 à 10 atomes de carbone, inclusivement;

m est un nombre entier valant de 0 à 15, inclusivement;

n est un nombre entier valant de 0 à 15, inclusivement;

X représente:

a) $—CH_2—$; ou

b) un groupe alkylène ramifié ayant de 2 à 15 atomes de carbone; ou

c) un groupe cycloalkylène, bicycloalkylène ou polycycloalkyène ayant de 4 à 10 atomes de carbone; ou

d) —O—

à l'exception des composés répondant à la formule ci-dessus dans laquelle $R_1$ et $R_2$ sont chacun un atome d'hydrogène et le radical

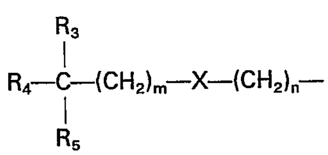

représente un groupe éthyle, n-propyle, isopropyle, n-butyle, tertiobutyle, isobutyl, n-décyle, pentadécyle ou cyclopentylméthyle, et à l'exception des esthers diméthylique de l'acide 3-hydroxy-3-propylglutarique et de l'acide 3-hydroxy-3-isopropylglutarique.

2. Composé selon la revendication 1, dans lequel X représente —$CH_2$—, à l'exception des composés répondant à la formule ci-dessus dans laquelle $R_1$ et $R_2$ sont chacun un atome d'hydrogène et le radical

$$R_4-\overset{\displaystyle R_3}{\underset{\displaystyle R_5}{\overset{|}{\underset{|}{C}}}}-(CH_2)_m-X-(CH_2)_n-$$

représente un groupe éthyle, n-propyle, n-butyle, n-décyle, pentadécyle ou cyclopentylméthyle, et à l'exception de l'esther diméthylique de l'acide 3-hydroxy-3-propylglutarique.

3. Acide 3-hydroxy-3-[10-(cyclohexyl)décyl]glutarique, composé selon la revendication 2.

4. Acide 3-hydroxy-3-[4-(cyclohexyl)butyl]glutarique composé selon la revendication 2.

5. Acide 3-hydroxy-3-[6-(cyclohexyl)hexyl]glutarique, composé selon la revendication 2.

6. Acide 3-hydroxy-3-[6-(cyclohexyl)octyl]glutarique, composé selon la revendication 2.

7. Acide 3-hydroxy-3-[11-(cyclohexyl)undécyl]glutarique, composé selon la revendication 2.

8. Acide 3-hydroxy-3-[13-(cyclohexyl)tridécyl]glutarique, composé selon la revendication 2.

9. Acide 3-hydroxy-3-[11,11-(dicyclohexyl)undécyl]glutarique, composé selon la revendication 2.

10. Acide 3-hydroxy-3-[10-(cycloheptyl)décyl]glutarique, composé selon la revendication 2.

11. Acide 3-hydroxy-3-[11-(cyclohexyl)pentadécyl]glutarique, composé selon la revendication 2.

12. Acide 3-hydroxy-3-[11-(cyclohexyl)dodécyl]glutarique, composé selon la revendication 2.

13. Ester monométhylique de l'acide 3-hydroxy-3-[10-(cyclohexyl)décyl]glutarique, composé selon la revendication 2.

14. Acide 3-hydroxy-3-[10-(1-méthylcyclohexyl)décyl]glutarique, composé selon la revendication 2.

15. Acide 3-hydroxy-3-[11-(1-méthylcyclohexyl)undécyl]glutarique, composé selon la revendication 2.

16. Acide 3-hydroxy-3-[11-(2-norbornyl)undécyl]glutarique, composé selon la revendication 2.

17. Acide 3-hydroxy-3-[10-(2-adamantyl)décyl]glutarique, composé selon la revendication 2.

18. Acide 3-hydroxy-3-[11-(1-adamantyl)undécyl]glutarique, composé selon la revendication 2.

19. Acide 3-hydroxy-3-(11-éthyltridécyl)glutarique composé selon la revendication 2.

20. Acide 3-hydroxy-3-(10,10-diméthylundécyl)glutarique, composé selon la revendication 2.

21. Acide 3-hydroxy-3-(11,11-diméthyldodécyl)glutarique, composé selon la revendication 2.

22. Acide 3-hydroxy-3-(12,12-diméthyltridécyl)glutarique, composé selon la revendication 2.

23. Composé selon la revendication 1 dans lequel X est un groupe cycloalkylène.

24. Acide 3-hydroxy-3-[6-(4-tertio-butylcyclohexyl)hexyl]glutarique, composé selon la revendication 23.

25. Acide 3-hydroxy-3-[10-(4-tertio-butylcyclohexyl)décyl]glutarique, composé selon la revendication 23.

26. Acide 3-hydroxy-3-[4-(décyl)cyclohexyl]glutarique, composé selon la revendication 23.

27. Composé selon la revendication 1, dans lequel X représente —O—.

28. Acide 3-hydroxy-3-[10-(cyclohexyloxy)décyl]glutarique, composé selon la revendication 27.

29. Acide 3-hydroxy-3-(10-hydroxydécyl)glutarique, composé selon la revendication 27.